Europäisches Patentamt

European Patent Office

Office européen des brevets

(11) Veröffentlichungsnummer: **0 374 446**

**A2**

# (12) EUROPÄISCHE PATENTANMELDUNG

(21) Anmeldenummer: 89120342.4

(22) Anmeldetag: 03.11.89

(51) Int. Cl.5: **A61K 6/08**

(30) Priorität: **18.11.88 DE 3839069**

(43) Veröffentlichungstag der Anmeldung:
**27.06.90 Patentblatt 90/26**

(84) Benannte Vertragsstaaten:
**BE CH DE FR GB IT LI LU NL SE**

(71) Anmelder: **ERNST MÜHLBAUER KG**
**Elbgaustrasse 248**
**D-2000 Hamburg 53(DE)**

(72) Erfinder: **Engelbrecht, Jürgen, Dr.**
**Petkumstrasse 18**
**D-2000 Hamburg(DE)**

(74) Vertreter: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

(54) **Verfahren zur Farbgestaltung und Versiegelung computergefräster Formteile für dentale Zwecke.**

(57) Die Erfindung betrifft ein Verfahren, mit dem computergefräste dentale Formkörper, insbesondere Kronen, Brücken, Inlays, Onlays, Facetten und künstliche Zähne farblich mit Hilfe von dünnen härtbaren Farbschichten gestaltet werden und anschliessend mit einer nahezu farblosen, durchscheinenden, glänzenden und abrasivfesten Schicht versiegelt werden.

EP 0 374 446 A2

## Verfahren zur Farbgestaltung und Versiegelung computergefräster Formteile für dentale Zwecke

Die Erfindung betrifft ein Verfahren zur Farbgestaltung und Versiegelung computergefräster Formteile für dentale Zwecke, insbesondere von computergefrästen Kronen und Brücken aus (Composite-) Kunststoffen, Metallen, Porzellanen oder Gusskeramiken.

Der übliche Weg zur Herstellung von Formteilen für dentale Zwecke, insbesondere von Kronen und Brücken, ist sehr kompliziert: Nach Abdrucknahme im Mund des Patienten mit Hilfe von speziellen Abdruckmassen werden Modelle der Zähne bzw. des Gebisses hergestellt. An diesen Modellen wird der Formkörper ganz oder teilweise aus Wachs oder ähnlichen provisorischen Modelliermaterialien geformt. Diese provisorischen Formkörper werden wiederum in aufwendigen Hochtemperaturverfahren durch Metall-, Keramik- oder Glasschmelzen substituiert und dann wiederum in weiteren Aufbrenn- und Auftrageverfahren zur fertigen Krone oder Brücke verarbeitet. Diese kann dann nach Tagen oder Wochen im Munde des Patienten eingesetzt werden.

Vor wenigen Jahren ist es nun möglich geworden, mit Hilfe einer "optischen" Abdrucknahme die Geometrie und Lage von Zähnen im Munde exakt numerisch zu erfassen. Aufgrund dieser Daten kann dann in weniger als einer Stunde mit Hilfe von computergesteuerten Fräsmaschinen eine Krone, Brücke oder andere Formteile hergestellt werden (EP 0091876, EP 0110797). Die so hergestellten Formteile sind extrem passgenau. Das Volumen, das der Zement bei der Einzementierung benötigt, ist von vornherein in definierter Schichtdicke mitberücksichtigt. Kronen und Brücken, die auf solche Weise angefertigt sind, sind technisch perfekt.

Ein grosser Nachteil dieser computergefertigten Formteile ist jedoch ihre nicht ausreichende Ästhetik. Während der natürliche Zahn eine Vielzahl von Farbspielen und Unregelmässigkeiten aufweist, muss das Material, aus dem verschiedenste Kronen unbekannter Form und Grösse gefräst werden sollen, farblich einheitlich sein. Zudem weisen solche Materialien, die von ihren mechanischen Eigenschaften, die für diese dentalen Formteile erforderlich sind, geeignet sind, selbst nach Politur eine deutliche Rauhigkeit auf, so dass eine äusserliche Verfärbungsgefahr sehr gross ist.

Aufgabe der vorliegenden Erfindung war daher, ein Verfahren zu finden, welches in einfacher und schneller Weise erlaubt, computergefräste Formteile für dentale Zwecke dauerhaft und ästhetisch so zu gestalten, dass Farbe, Form und Glanz an die natürlichen Zähne angepasst und dauerhaft erhalten werden können.

Es wurde nun gefunden, dass eine solche dauerhafte Farbgebung und eine abrasionsfeste, glänzende Oberflächenversiegelung der computergefrästen dentalen Formkörper erhalten wird, wenn zuerst die äussere und gegebenenfalls die innere Oberfläche des Formkörpers durch Aufbringen dünner, strahlenhärtbarer Farbschichten charakterisiert wird und dann die äussere Oberfläche durch Auftragen und Härten einer mässig dünnen, nahezu farblosen, durchscheinenden Schicht versiegelt wird.

Im folgenden werden Verfahrensweise und Materialien näher beschrieben.

Zur Restauration fehlender Zahnteile im Munde eines Patienten wird zunächst festgehalten, wie die Geometrie des fehlenden Teiles aussehen muss, damit Passgenauigkeit und Occlusionsverhalten stimmen. Dieses geschieht z.B. durch eine "optische Abdrucknahme" nach EP-0091876.

Anhand solchermassen erfassten numerischen Daten wird dann das benötigte Formteil aus einem zahnähnlichen gefärbten Werkstoff gefräst.

Der zahnähnlich gefärbte Werkstoff muss formstabil, mundverträglich und chemisch resistent sein. Werkstoffe solcher Art sind bekannt und werden für klassische Techniken wie z.B. zur Herstellung von Kronen, Brücken, Inlays, Onlays, Facetten oder Zähnen direkt oder in gegenseitiger Kombination verwendet. In Betracht kommen:

Kunststoffe, ungefüllt oder mit organischen oder anorganischen Füllstoffen verstärkt, meist auf Basis von polymerisierten (Meth-)Acrylestern, Polycarbonaten, Polymethanen oder Polyaminen; Porzellane, als wässriger Teig geformt und dann gebrannt; giessbare Glaskeramiken, giessbare Apatitkeramiken und Metalle, soweit sie mit o.g. Werkstoffen ganz oder teilweise überdeckt werden.

Vorzugsweise sollen die obengenannten Werkstoffe in 10-20 Zahn-Grundfarben, die etwa den Dentin-Farben nach bekannten Farbsystemen wie dem Vita-Farbsystem oder dem Biodent-Farbsystem entsprechen, eingefärbt sein. Steht aus ökonomischen Gründen nur eine Grundfarbe zur Verfügung, so sollte diese möglichst hell sein (beispielsweise Vita B 1, Biodent 11).

Bevor ein computergefrästes einfarbiges Formteil farblich mit Hilfe von härtbaren Farblacken gestaltet werden kann, muss gegebenenfalls eine dünne Schicht eines Haftvermittlers aufgetragen werden. Die Art des Haftvermittlers hängt von der chemischen Art des Werkstoffes ab, aus dem das Formteil gefräst ist. Werkstoffe z.B. aus glasgefüllten Composites, deren Harzbasis ähnlich der Harzbasis polymerisierbarer Kunststofffüllungsmaterialien ist, werden mit einer dünnen Schicht härtbarer Methacrylatharze versehen und ausgehärtet.

Keramik- und Glaskeramikformkörper werden vor der Farbcharakterisierung mit Silan-Haftvermittler behandelt. Geeignete Haftvermittler dieser Art sind Silane, die ungesättigte Gruppen tragen, wie z.B. Vinyltriethoxysilan oder Methacrylglycidyltriethoxysilan.

Ein weiterer Schritt muss noch vor der Farbgestaltung vorbereitet werden. Im Falle von etwas durchscheinenden bis hin zu fast transparenten Formkörpern muss gegebenenfalls die Farbe des Untergrundes, auf dem der Formkörper später im Munde des Patienten fixiert wird, mit berücksichtigt werden. Um solche Farbeffekte zu berücksichtigen, muss ein temporärer, genau passender Hintergrund ausserhalb des Mundes zur Verfügung stehen. Zudem muss dieser Hintergrund die Funktion eines Halters während der Behandlung des Formkörpers sowie der Aushärtung der Malfarben erfüllen.

Als geeignete Materialien für diese Funktion, im folgenden allgemein "provisorischer Stumpf" genannt, haben sich Materialien erwiesen, die sonst zur Anfertigung von provisorischen Kronen eingesetzt werden. Sie sind nach dem Mischen gut formbar, können an die Innenseite des Formkörpers gedrückt werden und nach Abbindung gut entformt werden. Ihr etwas elastisches Verhalten in dieser Abbindephase erleichtert das Abnehmen. Beispiele solcher handelsüblicher Materialien sind: Cronsin (Merz, Deutschland), Scutan, Protemp (Espe, Deutschland). Im Prinzip eignet sich jedoch jedes härtbare, vor der Härtung noch modellierbare Material.

Zur besseren Entformbarkeit und als Abstandhalter (Spacer) für den "Zement-Raum" wird zwischen Formkörper und "provisorischem Stumpf" eine dünne Schicht eines Wassergels (DE-OS 33 30 853) gegeben.

Ein "provisorischer Stumpf" kann selbstverständlich auch in mehr klassischer Weise durch Abdrucknahme des natürlichen "Stumpfes" im Munde und Herstellen eines Replicas aus Gips oder Modellierkunststoff erfolgen. Auch anhand der Computerdaten kann aus einem billigen Werkstoff ein "provisorischer Stumpf" gefräst werden.

Der "provisorische Stumpf" kann nun zwecks späterer Farbbetrachtung des Formkörpers mit der Farbe des später im Munde zu verwendenden Zementes bemalt werden. Vorzugsweise ist jedoch das Material des "provisorischen Stumpfes" selbst schon zahnfarben eingestellt.

Als optisches Kontakt-Medium zwischen Formkörper und "provisorischem Stumpf" kann eine Schicht leicht entfernbaren Wassergels dienen.

Die eigentliche Farbgestaltung des Formkörpers ist jetzt einfach durchzuführen:
Der Formkörper ist leicht mit Wassergel auf dem "provisorischen Stumpf" zu fixieren und zeigt nahezu exakt den farblichen Hintergrund, wie er am natürlichen Zahn erscheinen wird. Hatte der Werkstoff des Formkörpers nun schon eine Zahnfarbe, die der des Patienten im Munde weitestgehend entspricht, brauchen jetzt nur z.B. die Schneidebereiche, der Hals sowie die Interdentalräume mit intensiv gefärbten dünnen, härtbaren Farbpasten sehr dünn bemalt werden. Risse oder Fissuren können mit Hilfe von sehr dünn fliessenden, härtbaren Tintern eingezeichnet werden.

Hat der Werkstoff für den Formkörper eine nicht den natürlichen Zähnen des Patienten entsprechende Farbe, so kann diese durch Überziehen des gesamten oder eines wesentlichen Teils des Formkörpers mit einer zahnähnlich gefärbten, härtbaren Farbpaste der Zahnfarbe des Patienten angeglichen werden und dann wie vorgenannt farbcharakterisiert werden.

Die dünnen Intensivmalfarben und zahnfarbenen Malfarben sind z.B. eingefärbte, lichthärtbare, niedrig viskose Compositeformulierungen, vorzugsweise gefüllt mit feinstteiligen, anorganischen Füllstoffen (Mikrofiller), wie sie zur Charakterisierung von Füllungswerkstoffen und Kronen-und Brückenmaterialien im Handel erhältlich sind, wie z.B. Isosit-N Color (Ivoclar, Liechtenstein), Helio-Tint (Ivoclar), Dentacolor Intensiv- und Malfarben (Kulzer, Deutschland).

Als dünnfliessende Tinter für Fissuren und Rissmalereien sind vorzugsweise ungefüllte, eingefärbte, polymerisierbare Methacrylatharze geeignet.

Je nach Art der Initiatoren-Beimischung können die Intensiv-Zahn- oder Fissurenfarben z.B. durch Halogenlicht (Bereich von 600 nm bis 400 nm), Stroboskoplicht (Bereich von 500 nm bis 300 nm) oder UVA-Licht (Bereich von 400 nm bis 300 nm) gehärtet werden. Geeignete Initiatoren sind z.B.: alpha,beta-Diketone, Acrylphosphinoxide, Benzoinderivate, Benzophenone mit den entsprechenden Synergisten wie Aminen, Thioalkoholen, Thioxantone usw.. Initiatoren-Beimischungen können jedoch auch z.B. organische Peroxide, Benzpinakole, Diazoverbindungen u.a. sein, die bei thermischen Härteverfahren eingesetzt werden können.

Die bevorzugte Form der Härtung ist die Lichtaushärtung mit einer Stroboskoplampe hoher Leistung, wie z.B. einen Dentalcolor XS-Lichtgerät der Firma Kulzer.

Die Farbgestaltung der Oberfläche der computergefrästen Krone ist jedoch nicht unproblematisch. Einerseits sollen die aufgetragenen (Farb-)Schichten nicht zu dick sein, um nicht die äussere Geometrie des Formkörpers zu stark zu verändern, andererseits soll die Farbgestaltung z.B. einer Krone auch nach einigen Jahren der Abrasion im Munde im wesentlichen noch unverändert sein.

Erfindungsgemäss konnten diese Probleme durch Anwendung folgender Technik gelöst wer-

1. Die zur farblichen Charakterisierung gewählten Farbpasten werden sehr dünn und farbintensiv eingestellt und in Schichten von weniger als 0,1 mm aufgetragen. Das wesentliche Merkmal dieser Pasten ist die Fähigkeit, die gewünschte Farbe in geringer Schichtdicke zu erreichen, auf dem Formteil zu haften, mechanisch ausreichend fest und farbstabil zu sein. An die Abrasionsfestigkeit oder Polierbarkeit können geringere Anforderungen gestellt werden.

2. Die gesamte Aussenfläche des Formkörpers wird mit einer bis zu maximal 0,5 mm starken, hochabrasivfesten, dauerhaft hochglänzenden, farbstabilen und weitestgehend transparenten Schicht überzogen und damit versiegelt.

Die unter 1. genannten Farbpasten für die Charakterisierung sind, was das Material anbetrifft, nicht so sehr kritisch. Wichtig dabei ist nur, dass diese Farbpasten so mit Lichtaktivatoren eingestellt sind und so ausreichend belichtet werden, dass der überwiegende Teil der Schichtdicke ausreichend anpolymerisiert ist, damit beim anschliessenden Auftrag der Versiegelungsschicht keine Verlagerungen oder Verschmierungen der Farbschichten stattfinden. In diesem Stadium noch nicht optimal ausgehärtete Schichten werden dann zusammen mit der Versiegelungsschicht auspolymerisiert.

Die Anforderungen an Materialien, die für die Versiegelungsschicht geeignet sein sollen, sind nicht so einfach zu erfüllen:

Ein geeignetes Material muss niedrig viskos und leicht applizierbar sein, vorzugsweise mit einem Pinsel, soll dennoch aber einen hohen Anteil an Füllstoffen enthalten, um nach der Polymerisation einen geringen Schrumpf, eine hohe Härte, verbunden mit einer guten Hochglanzpolierbarkeit, aufzuweisen.

Ein geeignetes Material soll in gehärtetem Zustand hochabrasivfest und dennoch weitestgehend transparent sein.

Ein geeignetes Überzugsmaterial muss auch an sehr dünnen Stellen bestens auspolymerisierbar sein, so dass die Schicht nicht beschliffen werden muss, um durch Sauerstoffinhibition schlecht auspolymerisierte Schichten abzutragen.

Es hat sich nun jedoch gezeigt, dass alle diese Anforderungen mit härtbaren Composites erfüllt werden können, wenn diese einerseits als Füllstoffe mikrofeine amorphe Kieselsäure, sogenannte Mikrofiller, enthalten, und wenn sie andererseits nach der Polymerisation noch oberflächenvergütet werden. Composites, die mit Mikrofiller gefüllt sind, sind in den Patenten WO 81/01366, DE-OS 33 00 321, DE-PS 24 03 211 beschrieben. Wichtig ist, dass diese Füllstoffe silanisiert, bevorzugt (Meth-)acrylsilanisiert sind. Weiterhin bevorzugt werden diese mikrofeinen amorphen Kieselsäuren in ihrer ursprünglichen Form, d.h. nicht in Form von mit ihnen gefüllten Splitterpolymerisatfüllstoffen, eingesetzt. Weiterhin wichtig ist, dass eine grosse Menge von diesen Mikrofillern eingearbeitet werden kann, ohne dass die Viskosität der Paste zu hoch wird, um sie in dünnen Schichten aufzutragen. Als bevorzugte Form der Mikrofiller werden deshalb gesinterte Kieselgele, wie sie in DE-OS 33 00 321 beschrieben sind, eingesetzt. Besonders geeignete Ausführungen für die Versiegelung der Formkörper sind daher härtbare Composites, die zu 50% mit hochtemperaturgesintertem, methacrylsilanisiertem Kieselgel gefüllt sind. Diese Composites sind von dünner Konsistenz, leicht zu applizieren, zeigen eine hohe Härte, sind hochabrasionsfest und weisen eine sehr gute Hochglanzpolierbarkeit auf. Composites dieser Art werden im Handel z.B. als "Liquicoat" (Firma Merz, Deutschland) angeboten. Werden bei diesen Pasten bei der Herstellung die Pigmente weggelassen, sind sie nach Aushärten nahezu farblos und fast transparent.

Nachdem die Versiegelungsschicht in einer Stärke von bis zu 0,5 mm auf die äusseren Schichten des farbcharakterisierten Formteiles aufgetragen und gehärtet worden ist, erfolgt die obengenannte Oberflächenvergütung, die zu einer optimalen Oberflächenaushärtung führen soll.

Die Oberflächenvergütung kann darin bestehen, dass von vornherein unter inertem Schutzgas, wie z.B. Stickstoff, im Ultra-Hochvakuum oder in wässrigem oder Glycerin-Medium, gehärtet wird. Besonders einfach kann eine solche Oberflächenvergütung z.B. auf zwei Weisen zufriedenstellend erreicht werden:

Entweder man überschichtet die zuletzt applizierte und gehärtete Schicht des Versiegelungscomposites mit einer ca. 2 mm dicken Schicht eines Wassergels, wiederholt die Härtung und spült das Wassergel wieder mit Wasser ab. Die durch das Wassergel vor Sauerstoff geschützte Schicht ist jetzt sehr gut ausgehärtet.

Oder, und das ist die bevorzugte Ausführung, man überschichtet die zuletzt applizierte Schicht des Versiegelungscomposites mit einem speziellen, trocken härtenden, strahlenhärtbaren Lack, wie er z.B. für die Versiegelung von Prothesen, kieferorthopädischen Regulierungshilfen usw. im Handel angeboten wird (z.B. "Rezalite" Varnish, Firma Dynaflex, USA).

Der so applizierte strahlenhärtbare Lack ist nach der Aushärtung zwar nicht so abrasionsfest wie gefordert, aber seine sauerstoffschützende Wirkung wird schon bei einem Auftrag in einer Schichtstärke von 0,01 bis 0,02 mm erzielt. Nach einer solchen Anwendung sind die darunterliegenden Schichten optimal auspolymerisiert.

Nach Anwendung der Oberflächenvergütungstechnik ist die Farbgestaltung des dentalen Formteiles beendet und es kann im Munde des Patienten eingesetzt werden.

Die Farbe des Zementes, mit dem das fertige Formteil eingesetzt wird, kann nahezu farblos und transparent sein, wenn das Formteil genug lichtdurchlässig ist und es wünschenswert ist, den Farbton des darunterliegenden Zahnmaterials, z.B. des Stumpfes, auf den eine Krone gesetzt wird, durchscheinen zu lassen, um eine gute optische Tiefe zu erreichen.

In der Praxis bevorzugt werden jedoch mehr oder minder opake, definiert eingefärbte Zemente. Solche Zemente gestatten es, dem Zahnarzt oder Zahntechniker Tabellen zur Verfügung zu stellen, auf denen genau verzeichnet ist, welche Zemente und welche Malfarben er benutzen muss, um einen definierten Farbeindruck zu erreichen. Sollten trotz solcher Tabellen leichte Abweichungen auftreten, ist der Schaden nicht gross. So kann z.B. eine Krone noch vor der Aushärtung des Zementes wieder abgenommen, die Versiegelung weggeschliffen, neu charakterisiert und versiegelt und wieder einzementiert werden. Überhaupt besteht eine wesentlicher Vorteil des beschriebenen erfindungsgemässen Verfahrens darin, dass die gesamte Farbgestaltung der dentalen Formteile in der zahnärztlichen Praxis bei Anwesenheit des Patienten geschehen kann und dass Korrekturen leicht möglich sind. Alle diese Arbeiten können bei entsprechender Übung in 10 bis 20 Minuten durchgeführt werden.

Das erfindungsgemässe Verfahren soll anhand des folgenden Beispiels illustriert werden:

Beispiel

(Farbliche Gestaltung und Versiegelung einer computergefrästen Frontzahnkrone, die dem Farbton einer VITA-A 4-Farbe entsprechen soll)

Der Werkstoff, aus dem die gewünschte Krone mit Hilfe eines Computers gefräst werden soll, wurde folgendermassen hergestellt: 12,96 g strahlenhärtbares Composite DB 10 des Produktes "Dentacolor" (Firma Kulzer, Deutschland) wurden mit 5,29 g einer Mischung aus 37,5 Teilen Glasfaserabschnitten eines mittleren Durchmessers von 6 Mikron und einer mittleren Länge von 40 Mikron (Firma Schott Glaswerke, Deutschland), 8,2 Teilen einer Mischung aus Urethandimethacrylat, Triethylenglykoldimethacrylat, Bisphenol A Bisglycidylmethacrylat (1:1:1) sowie 0,2 Teilen Lauroylperoxid innig vermischt, in eine Würfelform von 10 x 10 x 10 mm gegeben und in einem Drucktopf bei 6 Atm. und 120°C in Glyerin 20 Minuten ausgehärtet.

Der ausgehärtete Werkstoff hatte etwa die Farbe Vita B 1. Der Würfel wurde in eine Matrix "Technovit" der Firma Kulzer eingebettet. Nach Aushärten wurden Würfelkörper und Einbettmasse so beschliffen, dass die Kombination für die Aufnahme einer Werkzeugmaschine in der richtigen Form vorlagen.

In dieser Form wurde aus dem Werkstoff anhand von numerischen Daten, die durch optisches Vermessen im Munde des Patienten gewonnen worden waren, eine Frontzahnkrone gefräst, die genau in Grösse und Form für den Zahnstumpf und für die Occlusionsverhältnisse im Munde des Patienten geeignet war. Die Schichtdicke dieser Krone lag zwischen 1 und 2,5 mm.

Ein "provisorischer Stumpf" wurde jetzt folgendermassen hergestellt: Die Innenseite der Krone wurde mit einer dünnen Schicht eines Wassergels (Superlux Surface Hardener, DMG, Hamburg) isoliert, mit angemischtem provisorischen Kronen- und Brücken-Material "Cronsin" in der Farbe "gelb" (Firma Merz) angefüllt und auf einem geeigneten Ständer fixiert. Nach Aushärten dieses Materials konnte die Krone leicht abgenommen werden. Der Stumpf wies, bis auf den Raum, den das Wassergel eingenommen hatte (Spacer), genau die Geometrie des Patientenstumpfes auf.

Während der folgenden Charakterisierungen wurde die Krone wieder mit Wassergel leicht auf dem "provisorischen Stumpf" befestigt. Die Krone zeigte jetzt, von aussen betrachtet, die Farbe bzw. die Farbspiele auf, die sie auch später auf dem natürlichen Stumpf nach Aufkleben mit Klebezement zeigen sollte. Bei den folgenden Arbeiten musste die gesamte Aussenfläche frei von Staub und Feuchtigkeit gehalten werden.

Die Charakterisierung erfolgte folgendermassen: Auf die gesamte Oberfläche der Krone, ausgenommen des Bereiches der Schneide, wird eine ca. 0,05 mm dicke Schicht einer Mischung aus "Flexo-Ceram Light Cure Tints" (Firma Elephant, Holland), und zwar aus einem Teil "Blue", zwei Teilen "Yellow" sowie einem Teil "Brown", aufgepinselt und in einem Dentacolor XS Gerät 90 Sekunden ausgehärtet.

Darauffolgend wurde der Schneidenbereich mit einer ca. 0,05mm dicken Schicht des Produktes "Superlux Esthetik" in der Farbe "blau transparent" (DMG, Hamburg) bemalt und der Halsbereich mit einer ca. 0,05 mm dicken Schicht einer Mischung von "Flexo-Ceram Light Cure Tints", ein Teil "Blue", ein Teil "Yellow", ein Teil "Brown", die mit Hilfe von Eisenoxidpigmenten Bayferrox 930 und Bayferrox 130 B (Bayer, Deutschland) auf die Farbe des Halses der Farbe A 4 des Vita-Zahnfarbenringes verstärkt wurde, belegt.

Nun wurde erneut im Dentacolor XS Gerät 90 Sekunden gehärtet. Die Krone verbleibt während all dieser Arbeiten auf dem "provisorischen Stumpf".

Das Aussehen der Krone stimmt jetzt ausreichend mit einem Modellzahn der Farbe Vita A 4 überein, welche für den Patienten gewünscht wurde.

Die gesamte Krone wurde jetzt mit einer 0,2 bis 0,4 mm dicken Schicht eines mit 50% gesinterten Kieselgels gefüllten, lichthärtenden Composites in uneingefärbter Form, welches unter der Bezeichnung "Superlux Esthetic-translucide" im Handel ist (DMG, Hamburg), mit Hilfe eines Pinsels überzogen und in einem Dentacolor XS Gerät 180 Sekunden ausgehärtet. Um die Oberfläche der gesamten Krone zu vergüten, wurde die Oberfläche mit Ethanol von anhaftendem Staub oder Spuren unpolymerisierten Harzes befreit und dann mit einer hauchdünnen Schicht (ca. 0,01 bis 0,02 mm) eines trockenpolymerisierenden, strahlenhärtbaren Lakkes, der unter der Bezeichnung "Rezalite Varnish" im Handel ist (Firma Dynaflex, USA), versehen. Nach nochmaligem Aushärten von 90 Sekunden im Lichthärtegerät (Dentacolor XS) war die Krone auf der Oberfläche sehr gut durchpolymerisiert und sehr kratz- und abrasionsfest.

Die Krone wurde nun im Munde des Patienten eingesetzt. Dazu wurde eine Mischung aus einem Teil "Flexo-Ceram Light Cure Opaque Brown" (Firma Elephant, Holand) und einem Teil "Flexo-Ceram Light Cure Opaque Yellow" (Firma Elephant, Holland) zusammengemischt und zwei Tropfen dieser Mischung mit einem Tropfen "Flexo-Ceram Inlay Dual Catalyst" (Firma Elephant,Holland) zu einem dualhärtenden Zement vermischt. Mit diesem Zement wurde die Krone in üblicher Weise auf den Zahnstumpf gesetzt, die Überschüsse entfernt und der Zement mit Halogenlicht des Gerätes "Translux" (Firma Kulzer) durch die Krone hindurch in vier Segmenten je 20 Sekunden ausgehärtet.

Das Ergebnis war eine Krone, die sich ästhetisch sehr zufriedenstellend in die Reihe der übrigen Zähne einreihte.

**Ansprüche**

1. Verfahren zur Farbgestaltung und Oberflächenversiegelung computergefräster dentaler Formkörper, dadurch gekennzeichnet, dass

a. auf den Formkörper niedrig viskose härtbare Farbschichten aufgebracht und gehärtet werden und

b. der Formkörper dann mit einer nahezu farblosen, durchscheinenden, härtbaren, glänzenden oder glanzpolierbaren und abrasionsfesten Schicht überzogen wird, die dann gehärtet wird.

2. Verfahren nach Anspruch 1, dadurch gekennzeichnet, dass die dentalen Formkörper aus Kunststoffen, Keramiken, Glaskeramiken oder Metallen oder aus Kombinationen aus diesen bestehen.

3. Verfahren nach Anspruch 1 oder 2, dadurch gekennzeichnet, dass die Kunststoffe, Keramiken und Glaskeramiken zahnähnlich gefärbt sind.

4. Verfahren nach Anspruch 1 oder 2, dadurch gekennzeichnet, dass das gefräste Metallformteil vor der Farbgestaltung ganz oder teilweise mit einem zahnfarbenen Opaker abgedeckt ist.

5. Verfahren nach Anspruch 1-4, dadurch gekennzeichnet, dass zwischen Formkörper und Farbschichten und/oder Oberflächenversiegelung ein Haftvermittler appliziert wird.

6. Verfahren nach Anspruch 1-5, dadurch gekennzeichnet, dass der Haftvermittler für Keramik oder Glaskeramik ein methacrylfunktionelles Silan ist.

7. Verfahren nach Anspruch 1-6, dadurch gekennzeichnet, dass die dentalen Formkörper Kronen, Brücken, Inlays, Onlays, Facetten oder künstliche Zähne sind.

8. Verfahren nach Anspruch 1-7, dadurch gekennzeichnet, dass die härtbaren Farbschichten ungefüllte oder gefüllte, pigmentierte Massen auf Basis radikalisch polymerisierbarer Monomere sind.

9. Verfahren nach Anspruch 1-8, dadurch gekennzeichnet, dass die Farbschichten dünner als 0,1 mm sind.

10. Verfahren nach Anspruch 1-9, dadurch gekennzeichnet, dass die härtbare Oberflächenversiegelung durch Aufbringen und Härten von gefüllten Massen auf Basis radikalisch polymerisierbarer Monomere erfolgt.

11. Verfahren nach Anspruch 10, dadurch gekennzeichnet, dass die Massen mindestens zu 40% organischen oder anorganischen Füllstoff verstärkt sind.

12. Verfahren nach Anspruch 10 und 11, dadurch gekennzeichnet, dass der Füllstoff in überwiegender Menge aus feinstteiligem, amorphem Siliciumdioxid besteht.

13. Verfahren nach Anspruch 1-12, dadurch gekennzeichnet, dass die gehärtete Oberflächenversiegelung Hochglanz zeigt oder dauerhafter Hochglanz durch Polieren erzielt wird.

14. Verfahren nach Anspruch 1-13, dadurch gekennzeichnet, dass die Schichtdicke der gehärteten Oberflächenversiegelung maximal 0,5 mm ist.

15. Verfahren nach Anspruch 1-14, dadurch gekennzeichnet, dass die schon polymerisierte Oberflächenversiegelungsschicht mit Hilfe von Vergütungsmassnahmen vollständig nachpolymerisiert wird.

16. Verfahren nach Anspruch 15, dadurch gekennzeichnet, dass als Vergütungsmassnahme die Oberflächenversiegelungsschicht unter Schutzgas, im Hochvakuum, unter Wasser eingetaucht, oder nach Auftrag einer Schicht Wassergel polymerisiert oder nachpolymerisiert wird.

17. Verfahren nach Anspruch 15, dadurch gekennzeichnet, dass als Vergütungsmassnahme die Oberflächenversiegelungsschicht mit einem sehr dünnen Film, der nicht empfindlich gegen Sauerstoffinhibition ist, bedeckt wird und dieser in sehr dünner Schicht auf- und damit nachpolymerisiert wird.

18. Verfahren nach Anspruch 1-17, dadurch gekennzeichnet, dass die radikalisch polymerisierbaren Monomere Ester von (Meth-)Acrylsäuren sind.

19. Verfahren nach Anspruch 1-18, dadurch gekennzeichnet, dass die Härtung eine Strahlenhärtung ist.

20. Verfahren nach Anspruch 19, dadurch gekennzeichnet, dass die Strahlenhärtung im Bereich von 600 nm bis 300 nm geschieht.

21. Verfahren nach Anspruch 1-20, dadurch gekennzeichnet, dass die Pasten zur Farbcharakterisierung und zur Oberflächenversiegelung eine solche Konsistenz aufweisen, dass sie mit einem Pinsel applizierbar sind.

22. Verfahren nach Anspruch 1-21, dadurch gekennzeichnet, dass die Farbgebung des dentalen Formkörpers durch die Farbe des zur Befestigung des Formkörpers im Munde des Patienten verwendeten Zementes oder durch Bemalung der Klebeflächen des Formkörpers mit Malfarben von innen unterstützt wird.

23. Verfahren nach Anspruch 1-22, dadurch gekennzeichnet, dass das Formteil zum Zwecke der Farbgestaltung auf einer Basis fixiert wird, die in Fläche, Farbe und Opazität der Klebefläche des Zahnteils im Munde des Patienten entspricht.

24. Verfahren nach Anspruch 23, dadurch gekennzeichnet, dass die Basis aus einem modellierbaren, härtbaren Material besteht, welches in Farbe und Opazität dem endgültig zu verwendenden Zement entspricht.

25. Verfahren nach Anspruch 23 oder 24, dadurch gekennzeichnet, dass die Basis gleichzeitig ganz oder teilweise als Halter oder Ständer beim Charakterisieren, Versiegeln und Aushärten dient.

26. Verfahren nach Anspruch 1-25, dadurch gekennzeichnet, dass zwischen Basis und Formteil eine leicht entfernbare Trenn- oder "Spacer"-Schicht gebracht wird.

27. Verfahren nach Anspruch 1-26, dadurch gekennzeichnet, dass als Trenn- oder "Spacer"-Schicht eine dünne Schicht eines Wassergels dient.